## Europäisches Patentamt

(19) **European Patent Office**

**Office européen des brevets**

(11) Veröffentlichungsnummer: **0 032 245**
**B1**

(12) # EUROPÄISCHE PATENTSCHRIFT

(45) Veröffentlichungstag der Patentschrift:
**13.04.83**

(51) Int. Cl.³: **C 07 C 69/96**, C 07 C 68/02

(21) Anmeldenummer: **80108247.0**

(22) Anmeldetag: **30.12.80**

(54) **Verfahren zur Herstellung von aromatischen Chlorformiaten.**

(30) Priorität: **09.01.80 DE 3000524**

(43) Veröffentlichungstag der Anmeldung:
**22.07.81 Patentblatt 81/29**

(45) Bekanntmachung des Hinweises auf die Patenterteilung:
**13.04.83 Patentblatt 83/15**

(84) Benannte Vertragsstaaten:
**BE DE FR**

(56) Entgegenhaltungen:
**US-A-3 170 946**
**US-A-3 211 775**
**US-A-3 211 776**

(73) Patentinhaber: **BASF Aktiengesellschaft,
Carl-Bosch-Strasse 38, D-6700 Ludwigshafen (DE)**

(72) Erfinder: **Decker, Martin, Dr., Maria-Stuart-Strasse 21,
D-6700 Ludwigshafen (DE)**
Erfinder: **Neumayr, Franz, Dr., Im Eiertal 8,
D-6719 Weisenheim (DE)**
Erfinder: **Jaeger, Peter, Dr., Kranichstrasse 9,
D-6700 Ludwigshafen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Verfahren zur Herstellung von aromatischen Chlorformiaten

Die Erfindung betrifft ein Verfahren zur Herstellung von aromatischen Chlorformiaten durch Umsetzung von Phenolen mit Phosgen in Gegenwart quartärer Ammoniumsalze.

Bekanntlich lassen sich Chlorformiate von Phenolen durch Umsetzung von Phenolen mit Phosgen in Gegenwart stöchiometrischer Mengen eines tertiären Amins oder eines Alkalihydroxids als Salzsäureakzeptor herstellen. Diese Verfahren haben den Nachteil, daß beträchtliche Mengen Diarylcarbonate als Nebenprodukt entstehen. Die Verwendung eines Amins macht außerdem dessen Rückgewinnung mit Alkalilauge erforderlich.

Aromatische Chlorformiate lassen sich auch durch Umsetzung des Phenols mit Phosgen in Gegenwart einer katalytischen Menge des tertiären Amins bei erhöhter Temperatur gewinnen. Bei diesem Verfahren wird üblicherweise ein inertes Lösungsmittel in der 3- bis 4fachen Menge des Phenols eingesetzt. Man hat hierbei nicht nur das Lösungsmittel zurückzugewinnen, es ist auch erforderlich, das Rohprodukt mit verdünnter Salzsäure zu waschen, um aminfreie Produkte zu erhalten. Ferner muß das Produkt destilliert werden, um Diarylcarbonat und nicht umgesetztes Phenol abzutrennen.

Aus der US-PS 3 255 230 ist ein Verfahren bekannt, bei dem aromatische Dialkohole, wie Bisphenol A oder Hydrochinon in einem inerten Lösungsmittel und in Gegenwart von quartären Ammoniumsalzen als Katalysator und bei relativ hoher Phosgenkonzentration umgesetzt werden. Ein Alkylrest des quartären Ammoniumsalzes soll mindestens 10 bis 30 C-Atome enthalten. Die Verwendung eines Lösungsmittels sowie die niedrige Raum-Zeit-Ausbeute sind bei diesem Verfahren nachteilig.

In der US-PS 3 211 774 und der DE-AS 2 131 555 werden ähnliche Verfahren beschrieben, bei denen Dimethylformamid oder andere Dialkylformamide als Katalysatoren eingesetzt werden. Diese Katalysatoren haben den Nachteil, daß sie bei den erforderlichen Reaktionstemperaturen zur Zersetzung neigen und ihre Wirksamkeit verlieren. Außerdem ist bei diesen Verfahren die Raum-Zeit-Ausbeute gering, ferner entstehen Diarylcarbonate als Nebenprodukte.

Es wurde nun gefunden, daß man bei der Herstellung von aromatischen Chlorformiaten durch Umsetzung von Phenolen mit Phosgen in Gegenwart eines quartären Ammoniumsalzes als Katalysator besonders vorteilhafte Ergebnisse erzielt, wenn man den Katalysator und 2 bis 10 Gew.-% des herzustellenden Chlorformiates vorlegt und zu dieser Vorlage das Phosgen und das Phenol gibt, wobei man die Umsetzung in Abwesenheit eines Lösungsmittels durchführt.

Nach dem erfindungsgemäßen Verfahren erhält man die Chlorformiate auf besonders einfache Weise in hoher Raum-Zeit-Ausbeute und Reinheit.

Als Phenole kommen ein- und mehrwertige Alkohole in Betracht, deren Hydroxylgruppen an C-Atomen eines aromatischen Kerns gebunden sind, der noch andere Substituenten, wie Halogenatome, Alkylreste, Alkoxigruppen, Nitrogruppen oder ungesättigte Kohlenwasserstoffreste enthalten kann. Geeignete Phenole sind beispielsweise Phenol, p-Kresol, $\alpha$-Naphthol, o-sec.-Butylphenol, m-tert.-Butylphenol.

Als quartäre Ammoniumsalze kommen z. B. solche in Betracht, in denen die vier N-ständigen organischen Reste insgesamt 4 bis 15 C-Atome aufweisen, wie

Tetramethylammoniumchlorid,
Triäthylbenzylammoniumhydroxid,
Trimethylbenzylammoniumchlorid,
N,N-Dimethylpiperidiniumchlorid,
Tetraäthylammoniumchlorid,
N,N-Dimethylpyrrolidiniumchlorid und
N,N-Dimethylmorpholiniumchlorid.

Die Verwendung der Katalysatoren Trimethylbenzylammoniumchlorid und N,N-Dimethylpiperidiniumchlorid ist von besonderem technischem Interesse. Man wendet die Katalysatoren in Mengen von 0,5 bis 3, vorzugsweise 0,8 bis 1,5 Mol%, bezogen auf umzusetzendes Phenol an. Die Gesamtmenge des Katalysators wird bei dem erfindungsgemäßen Verfahren im Reaktionsgefäß vorgelegt.

Phosgen wird in mindestens stöchiometrischen Mengen eingesetzt. Vorzugsweise wendet man einen Überschuß bis zu 20 Mol%, bezogen auf das Phenol an.

Nach dem erfindungsgemäßen Verfahren werden 2 bis 10 Gew.-% des herzustellenden Chlorformiates im Reaktionsgefäß zusammen mit dem Katalysator vorgelegt. Im allgemeinen genügen 2 bis 5 Gew.-%, bezogen auf die Gesamtmenge des in einem Ansatz schließlich gebildeten Chlorformiates.

Man nimmt die Umsetzung der Phenole mit Phosgen zur Herstellung der Chlorformiate bei Temperaturen von 50° C bis 150° C, vorzugsweise von 90 bis 110° C vor. Zweckmäßigerweise erwärmt man die Vorlage aus Katalysator und Chlorformiat auf Temperaturen von 90 bis 110° C. Es kann auch zweckmäßig sein, einen Teil des Phosgens vor der Zugabe des Phenols zur Vorlage zu geben. Ein Überschuß an Phosgen kann sich schon zu Beginn der Umsetzung vorteilhaft auswirken. Im allgemeinen werden Phosgen und Phenol gleichzeitig zum Reaktionsgemisch gegeben. Nach Reaktionsende wird überschüssiges Phosgen, z. B. durch Einleiten von Stickstoff ausgeblasen.

Man erhält so unmittelbar Umsetzungsgemische in denen der Katalysator ausgefallen ist. Die Chlorformiate, die bei dem erfindungsgemäßen Verfahren in hoher Ausbeute gebildet werden, lassen sich durch einfaches Abfiltrieren vom Katalysator abtrennen. Der Katalysator kann beliebig oft für weitere Ansätze verwendet werden.

Bei einer besonders vorteilhaften technischen Durchführung des Verfahrens verfährt man so, daß man das gebildete Chlorformiat, auf dem sich der ausgefallene Katalysator als eine dünne schwimmende Schicht abscheidet, durch Ablassen der unteren Schicht, die praktisch katalysatorfrei ist, isoliert. Man läßt bei dieser Arbeitsweise etwa 5 bis 10% des gebildeten Chlorformiates mit dem aufschwimmenden Katalysator im Reaktionsgefäß zurück und beginnt mit dieser Vorlage durch Zugabe von Phosgen und Phenol die Umsetzung einer neuen Charge.

Überraschenderweise werden bei dem erfindungsgemäßen Verfahren die aromatischen Chlorformiate in sehr guter Raum-Zeit-Ausbeute und hoher Reinheit erhalten, so daß sie ohne vorherige Reinigung für Folgeprodukte eingesetzt werden können. die Reinheit der Rohprodukte liegt in der Regel über 99% und die Ausbeute über 98 Mol%.

### Beispiel 1

In einem Rührbehälter mit Begasungsvorrichtung werden 146 Gew.-Teile Trimethyl-benzylammoniumchlorid als Katalysator und 171 Gew.-Teile p-Kresylchlorformiat vorgelegt. Das Gemisch wird auf 95°C vorgewärmt, dabei werden gleichzeitig 50 Gew.-Teile Kohlenoxidchlorid eingeleitet. Danach werden im Temperaturbereich zwischen 95 und 105°C je Stunde 324 Gew.-Teile p-Kresol und gleichzeitig 330 Gew.-Teile Kohlenoxidchlorid der Mischung zugeführt. Insgesamt werden 2592 Gew.-Teile p-Kresol und 2710 Gew.-Teile Kohlenoxidchlorid umgesetzt. Nach der Beendigung des Zulaufs hält man die Temperatur der Reaktionsmischung noch 2 Stunden auf 100°C. Anschließend wird durch Einleiten von Stickstoff überschüssiges Kohlenoxidchlorid ausgeblasen. Danach ist der Katalysator praktisch quantitativ ausgefallen und wird durch Filtration vom Produkt getrennt. Er kann beliebig oft für weitere Ansätze verwendet werden. Es werden nach Abzug des vorgelegten Produktes 4060 Gew.-Teile rohes p-Kresylchlorformiat mit einer Farbzahl von 2 bis 3 (Jodskala) und mit einer Reinheit von 99,7% erhalten. Die Ausbeute beträgt 98,9 Mol%.

### Beispiel 2

In einem Rührbehälter werden 37 Gew.-Teile Trimethyl-benzyl-ammoniumchlorid, welche aus dem Versuch in Beispiel 3 zurückgewonnen wurden, zusammen mit 16 Gew.-Teilen Phenylchlorformiat vorgelegt. Die Mischung wird auf 95°C erwärmt. Dann werden im Temperaturbereich zwischen 95 und 102°C je Stunde 94 Gew.-Teile Phenol und 120 Gew.-Teile Kohlenoxidchlorid eingetragen. Insgesamt werden 188 Gew.-Teile Phenol und 240 Gew.-Teile Kohlenoxidchlorid umgesetzt. Zur Nachreaktion hält man die Temperatur noch 1 Stunde auf 105°C. Anschließend wird mit Stickstoff überschüssiges Kohlenoxidchlorid ausgeblasen. Man arbeitet wie in Beispiel 1 beschrieben auf und erhält 329 Gew.-Teile rohes 98,6%iges Phenylchlorformiat mit einem Gehalt an Diphenylcarbonat von 0,9 Gew.-%. Die Ausbeute beträgt 98,7 Mol%.

### Beispiel 3 (Vergleichsbeispiel)

In einem Rührbehälter werden 188 Gew.-Teile Phenol und 37 Gew.-Teile Trimethyl-benzylammoniumchlorid auf 115°C erwärmt. Dann werden innerhalb von 2,5 Stunden 220 Gew.-Teile Kohlenoxidchlorid eingeleitet. Zur Vervollständigung der Reaktion hält man noch 1 Stunde auf 105°C. Dann wird mit Stickstoff das überschüssige Kohlenoxidchlorid ausgeblasen. Der ausgefallene Katalysator wird mit einer Nutsche abgesaugt. Es werden 306 Gew.-Teile klares, helles Filtrat erhalten, das nach geaschromatographischer Analyse 97,0% Phenylchlorformiat und 2,8% Diphenylcarbonat enthält. Die Ausbeute beträgt 94,9 Mol% Phenylchlorformiat.

### Beispiel 4

In einem Rührbehälter werden 37,4 Gew.-Teile Trimethylbenzylammoniumchlorid und 104 Gew.-Teile 1-Naphthylchlorformiat auf 105°C erhitzt. In diese Mischung werden stündlich 144 Gew.-Teile 1-Naphthol und 120 Gew.-Teile Kohlenoxidchlorid im Temperaturbereich zwischen 95 und 105°C eingetragen. Insgesamt werden 1080 Gew.-Teile 1-Naphthol und 860 Gew.-Teile Kohlenoxidchlorid innerhalb von 7,5 Stunden zur Reaktion gebracht. Danach wird die Reaktionsmischung noch 1,5 Stunden bei 100°C gehalten. Anschließend wird das überschüssige Kohlenoxidchlorid mit Stickstoff ausgeblasen. Nach der Abtrennung des Katalysators durch Filtration werden 1545 Gew.-Teile rohes 1-Naphthylchlorformiat mit 99,4%iger Reinheit erhalten. Die Ausbeute beträgt 99,7 Mol%.

### Beispiel 5

In einem Rührreaktor mit 4000 Volumenteilen Fassungsvermögen, der mit einem Rückflußkühler ausgestattet ist, werden 150 Gew.-Teile o-sec-Butylphenylchlorformiat und 33,4 Gew.-Teile Trimethylbenzylammoniumchlorid vorgelegt und auf 95 bis 100°C erhitzt. Der Rückflußkühler ist auf −70°C gekühlt. In die Mischung werden stündlich im Temperaturbereich zwischen 95 und 105°C 150 Gew.-Teile o-sec-Butylphenol und 120 Gew.-Teile Phosgen eingeleitet. Insgesamt werden dem Rührreaktor 2850 Teile o-sec-Butylphenol und 2150 Teile Phosgen zugeführt. Danach hält man die Temperatur des Reaktionsgemisches noch 3 Stunden auf 100°C. Schließlich wird das noch vorhandene Phosgen mit Stickstoff ausgeblasen und der Katalysator mit einem Filter abgetrennt. Man erhält 4010 Gew.-Teile farbloses o-sec-Butylphenylchlorformiat, dessen Reinheit nach Gaschromatographie und Chloranalyse über 99,9% beträgt. Die Ausbeute beträgt 99,32 Mol%.

Beispiel 6

In einem Rührreaktor, der mit einem auf −70°C gekühlten Rückflußkühler versehen ist, werden 85 Gew.-Teile p-Kresylchlorformiat und 13,5 Gew.-Teile N,N-Dimethylpiperidiniumchlorid vorgelegt. Man erwärmt die Mischung auf 100 bis 103°C. Bei dieser Temperatur werden dann stündlich 108 Gew.-Teile p-Kresol und 110 Gew.-Teile Phosgen in die Mischung eingeleitet. Es werden insgesamt 998 Teile p-Kresol und 1140 Teile Phosgen zur Reaktion gebracht. Danach wird noch 2 Stunden bei 102°C gerührt und dann mit Stickstoff überschüssiges Phosgen ausgeblasen. Nach der Entfernung des Katalysators durch Filtration werden 1651 Gew.-Teile p-Kresylchlorformiat erhalten, was einer Ausbeute von 99,4 Mol% entspricht. Die Reinheit des Produktes beträgt nach Gaschromatographie 99,96%.

Patentansprüche

1. Verfahren zur Herstellung von aromatischen Chlorformiaten durch Umsetzung von Phenolen mit Phosgen in Gegenwart eines quartären Ammoniumsalzes als Katalysator, dadurch gekennzeichnet, daß man den Katalysator und 2 bis 10 Gew.-% des herzustellenden Chlorformiates vorlegt und zu dieser Vorlage das Phosgen und das Phenol gibt, wobei man die Umsetzung in Abwesenheit eines Lösungsmittels durchführt.

2. Verfahren nach Anspruch 1, dadurch gekennzeichnet, daß man nach der Umsetzung das gebildete Chlorformiat durch Filtration vom Katalysator abtrennt.

3. Verfahren nach Anspruch 1, dadurch gekennzeichnet, daß man als Katalysator Trimethylbenzylammoniumchlorid oder Dimethylpiperidiniumchlorid verwendet.

Claims

1. A process for the preparation of aromatic chloroformates by reacting phenols with phosgene in the presence of a quaternary ammonium salt as catalyst, wherein the catalyst and 2 to 10% by weight of the chloroformate to be prepared are first introduced into the reactor, and then the phosgene and phenol are added thereto, the reaction being carried out in the absence of a solvent.

2. A process as claimed in claim 1, wherein, following the reaction, the chloroformate formed is separated from the catalyst by filtration.

3. A process as claimed in claim 1, wherein trimethylbenzylammonium chloride or dimethylpiperidinium chloride is used as the catalyst.

Revendications

1. Procédé de préparation de chloroformiate aromatiques par réaction de phénols avec le phosgène en présence d'un sel d'ammonium quaternaire qui sert de catalyseur, caractérisé en ce que l'on mélange au début le catalyseur et 2 à 10% en poids du chloroformiate à préparer et on ajoute le phosgène et le phénol à ce mélange en effectuant la réaction en l'absence de solvant.

2. Procédé selon la revendication 1, caractérisé en ce que, après la réaction, on sépare le chloroformiate formé du catalyseur par filtration.

3. Procédé selon la revendication 1, caractérisé en ce que l'on utilise en tant que catalyseur le chlorure de triméthylbenzyl-ammonium ou le chlorure de diméthylpipéridinium.